(19) Europäisches Patentamt / European Patent Office / Office européen des brevets

(11) **EP 2 332 463 A1**

(12) **EUROPEAN PATENT APPLICATION**

(43) Date of publication:
**15.06.2011 Bulletin 2011/24**

(51) Int Cl.:
*A61B 5/053* [(2006.01)]   *A61B 5/11* [(2006.01)]
*A61B 19/00* [(2006.01)]

(21) Application number: **09450236.6**

(22) Date of filing: **14.12.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicants:
• **Technische Universität Graz**
  **8010 Graz (AT)**
• **Forschungsholding TU Graz GmbH**
  **8010 Graz (AT)**

(72) Inventors:
• **Scharfetter, Hermann**
  **8045 Graz (AT)**
• **Gürsoy, Doga**
  **8010 Graz (AT)**

(74) Representative: **Patentanwaltskanzlei**
**Matschnig & Forsthuber OG**
**Siebensterngasse 54**
**1071 Wien (AT)**

(54) **Device and method magnetic induction tomography**

(57)    A method and an apparatus for magnetic induction tomography, where an object having inhomogeneous electrical properties is subjected to alternating magnetic fields by means of at least one transmitter coil (ES1, ES2, ES3), picking up alternating voltage signals which contain information on the electrical conductivity and its distribution in the object by means of at least one receiving coil (SP1, SP2, SP3), and reconstructing an image of the electrical properties within the object from the received signals, having an imaginary and a real part, by using their different phases and amplitudes, whereby a set of markers (MAR) is placed on the surface of the object under investigation, the markers being selected from the group of markers producing a signal which is clearly distinguishable from that of the object, measurements are performed to reconstruct the image of the object itself on the one hand and reconstructing the image and location of the markers (MAR) on the other hand and filtering out the movements of the outer object boundary in order to eliminate artifacts due to such movements. Each marker (MAR) may be an active marker consisting of a loop (LOO), the wire being electrically conducting, and a remotely controlled switch (SWI), adapted to open and close the loop.

Fig. 5

**Description**

Field of the invention and description of prior art

The present invention relates to a Method for magnetic induction tomography, where an object having inhomogeneous electrical properties is subjected to alternating magnetic fields with at least one excitation frequency by means of at least one transmitter coil, picking up alternating voltage signals which contain information on the electrical conductivity and its distribution in the object by means of at least one receiving coil , electrically ore mechanically locating the at least one receiving coil at different reception locations and reconstructing an image of the electrical properties within the object from the received signals, having an imaginary and a real part, by using their different phases and amplitudes.

Magnetic induction tomography (MIT) is an imaging modality which, very much like electrical impedance tomography (EIT), aims at the mapping of the complex electrical conductivity $\kappa = \sigma + j\omega\varepsilon_r\varepsilon_0$ inside an object, e. g. a human body. $\omega$, $\sigma$ and $\varepsilon$ are the angular frequency, electrical conductivity and permittivity, respectively. MIT is therefore closely related to electrical bioimpedance. In contrast to EIT, however, the interrogating currents are not injected via electrodes but instead induced by alternating magnetic fields. Furthermore the received quantity is not the electric potential at the object surface but rather the vector of voltages induced by the total magnetic field in pickup coils.

[Literature on this: Griffiths H., Magnetic induction tomography. Meas. Sci. Technol. 26: 1126 -1131. Korzhenevskii A. V., and V. A. Cherepenin. Magnetic induction tomography. J. Commun. Tech. Electron. 42; 469 - 474,1997].

The document WO 2008/011649 A1, which is considered as a representative state of the art refers to a method and a device for MIT, whereby a measurement is carried out using at least two different frequencies and an additional perturbation of the coils and/or the field geometry so as to determine a correction factor with which spurious signals generated by changes of the geometry and amplifier drift during the object measurement can be substantially eliminated.

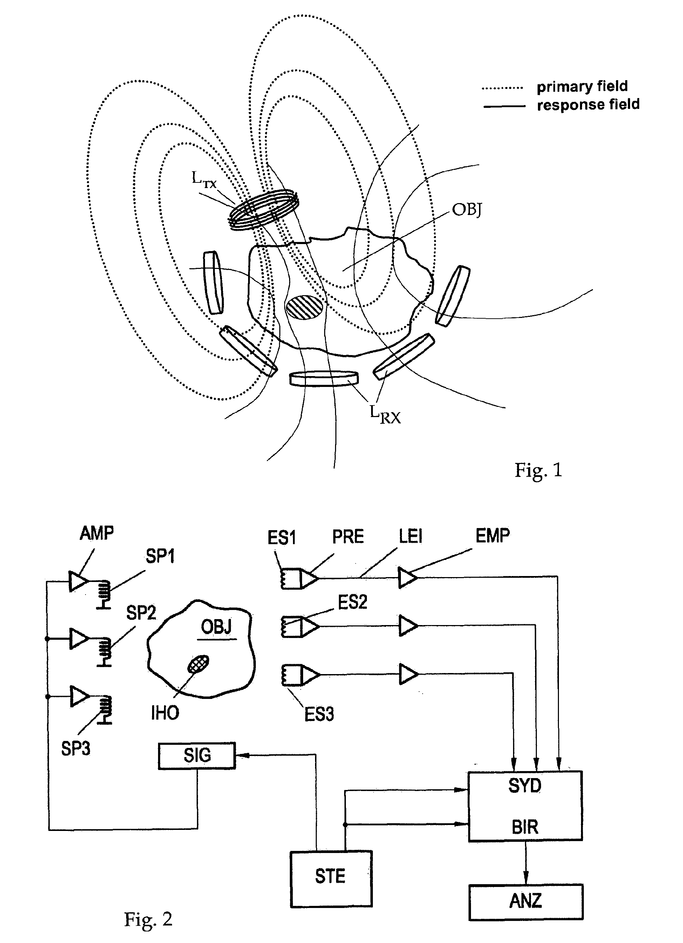

The principle of MIT is illustrated in Fig. 1 where the magnetic field is produced with a transmitter coil $L_{TX}$ and coupled to the body OBJ under investigation. The resulting total field is sensed by an array of receiver coils $L_{RX}$.

Figure 2 shows schematically an object OBJ to be investigated, having an inhomogeneity IHO which has a conductivity different from the remainder of the object, for example, a lesion inside a part of the body such as the brain or a female breast.

Three transmitter coils SP1, SP2 and SP3 are arranged at various positions outside the object to be investigated, but as close as possible thereto, but the number of transmitter coils can also be substantially higher according to the desired resolution and the type of object. These transmitter coils are supplied with AC current, originating from a signal generator SIG, having amplifiers AMP connected ahead thereof for each transmitter coil. Three receiver coils ES1, ES2, ES3 are located in the area of the transmitter coils here but can also be arranged at completely different positions. A pre-amplifier PRE is provided for each of the receiver coils and these pre-amplifiers are connected via shielded lines LE1 to further amplifiers EMP whose outputs are supplied to a synchronous detector SYD. The synchronous detector SYD receives the necessary synchronous signal from the sine generator SIG. An image reconstruction BIR also takes place in the unit with the synchronous detector and its output signal can then be passed to a display ANZ such as a screen, a printer etc. The synchronous detector SYD, the amplifiers AMP and the image reconstruction BIR are controlled by a control unit STE. A coil designated as REF is used to obtain a reference signal.

Since the signals to be evaluated which are picked up by the receiver coils are in fact many orders of magnitude smaller than the excitation signals of the transmitter coils, care is initially taken to ensure that the fields of the transmitter coils do not act directly on the receiver coils. For this purpose, the receiver coils may be configured as so-called gradiometer coils which can additionally be arranged orthogonally in relation to the transmitter coils. Such gradiometer coils are in principle insensitive to other fields as long as these fields are homogeneous since the same voltage but with opposite sign is induced in each coil half. Since neither the receiver coil geometry is perfect nor are the interference fields which occur actually homogeneous, appreciable spurious signals occur however, partly e. g. from long-to-short-wavelength transmitters. The processing by a synchronous detector in a known manner can considerably reduce the perturbation level here.

After preamplification the received voltages are usually fed to a synchronous demodulator where the real and imaginary parts are determined with respect to a reference signal. Alternatively direct phase detectors may also been used as the phase angle is roughly proportional to the imaginary part of the signal unless the primary voltage is suppressed by zero flow methods. Synchronous demodulation is nowadays most effectively done digitally after A/D conversion. In the case of simultaneous multifrequency operation FFT-based signal processing is a very efficient way to demodulate the signals.

The signals received in the receiver coils ES1, ES2 and ES3 depend, inter alia, on the distribution of the electrical conductivity inside the object OBJ to be investigated and it has been shown that tissue variations in the breast tissue, for example, lead to conductivity variations which are sufficiently large to allow a mammographic representation following evaluation in a microprocessor of the image processing DVA. Details need not be discussed here since these can be found, for example, in the citation already mentioned.

A basic presentation on the multi-frequency modification MIT can be found in [Hermann Scharfetter, Roberto Casanas and Javier Rosell, "Biological Tissue Characterization by Magnetic Induction Spectroscopy (MIS): Requirements and Limitations", IEEE Trans. Biomed. Eng. 50, 870 - 880, 2003].

The sensitivity of a coil system can be expressed as the voltage change $\Delta V$ in the transmitter coil due to a small change $\Delta \kappa$ in the space $\Omega$ filled with the electromagnetic field. Two parameters are important for the resolution of a certain $\Delta \kappa$: The relative voltage change $\Delta V/V0$ and the SNR $\Delta V/std(V)$, where $std(V)$ is the RMS value of the receiver noise voltage. The first quantity determines the required dynamic range and the second one the detectability.

A general expression for calculating the sensitivity can be derived from the law of reciprocity for a linear two-port-system with two ports fed by a current I [see: Mortarelli J. R. A., Generalization of the Geselowitz Relationship Useful in Impedance Plethysmographic Field Calculations IEEE Trans. Biomed. Eng. 1980; 27: 665 - 667]

$$\Delta V = \frac{1}{I}\left[\Delta \kappa \int_{\Omega}(\nabla \Phi + j\omega \mathbf{A}_{\Phi})\cdot(\nabla \Psi + j\omega \mathbf{A}_{\Psi})d\Omega + j\omega \Delta \mu \int_{\Omega}\mathbf{H}_{\Phi}\cdot\mathbf{H}_{\Psi}d\Omega\right] \qquad (1)$$

with $\Phi$, $\mathbf{A}_{\Phi}$: electrical scalar potential and magnetic vector potential when feeding the system from port A; $\Psi$, $\mathbf{A}_{\Psi}$: electrical scalar potential and magnetic vector potential when feeding the system from port B; $\mathbf{H}_{\Phi}$, $\mathbf{H}_{\Psi}$: the corresponding magnetic field intensity vectors.

Beside this so called adjoint method (the fields labeled with $\Psi$ and $\Phi$ are mutually adjoint fields) there is also the possibility of a direct calculation of the sensitivity, [see Gencer NG, Tek MN. Electrical conductivity imaging via contactless measurements. IEEE Trans Med Imag. 1999; 18: 617 - 27]. The general solution of eq. (1) requires elaborate numerical methods. For a raw estimation of the expected sensitivities, however, some simple analytical or semi-analytical solutions can be derived for special cases. A closed solution for a cylindrical sample with radius R and thickness t (t << 2a) positioned coaxially halfway between two small coils (assumed to be magnetic dipoles) with distance 2a was published in [IEEE Trans Biomed Eng. 2003; 50: 870 - 80, see above]. The relative voltage change can be expressed by eq. (2).

$$\frac{\Delta V}{V_0} = \frac{a^3 t}{2}\left\{(\mu_r - 1)\frac{R^2(8a^2 - R^2)}{(a^2 + R^2)^4} - j(\sigma + j\omega\varepsilon_0\varepsilon_r)\omega\mu_0\left[\frac{1}{a^2} - \frac{a^2 + 2R^2}{(a^2 + R^2)^2}\right]\right\} \qquad (2)$$

$\mu_r$ denotes the relative permeability of the material. Eq. (2) is valid only if the alteration of the excitation field by the response field is negligible and if $\mu_r$ is close to 1. This is the case if the penetration depth of the electromagnetic field is much larger than the thickness of the object which is true for biological objects up to several tens of MHz. One must bear in mind that such simplified models do not produce the correct sensitivity maps for the typical measureands found in biomedical applications. In the latter the conductive perturbations are embedded in a conducting background thus the sensitivity maps are completely different. However, assuming small spherical perturbations within a cylindrical background with conductivities close to the physiological range the absolute signal changes remain in the same order of magnitude as if the perturbation occurred in the empty space. Therefore eq. (2) still gives at least a rough guess of what can be expected: The imaginary part of $\Delta V/V_0$ is essentially proportional to changes of the conductivity $\sigma$ and the radian frequency $\omega$. The real part is essentially proportional to the relative permittivity $\varepsilon_r$ and to $\omega^2$. Considering that for biological tissue at low frequencies (10 Hz-1 kHz) $\varepsilon_r$ can be $10^4$ - $10^6$, its contribution can reach the same order of magnitude as that of the conductivity, at moderate frequencies.

The real part is also proportional to $\mu_r$ but this contribution does not depend on the frequency. The dielectric contribution can be neglected at low frequencies (some kHz) due to its dependence on $\omega^2$ if the permittivity is not excessively high. Moreover, below a certain frequency, also changes of the imaginary part fall below the noise level of the detectors due to the dependence on $\omega$. In this case changes of the permeability become entirely predominant in the signal.

If a highly conducting, non-magnetic material is introduced into the coil fields, eq. (2) looses its validity completely and the $\Delta V/V_0$ becomes nearly entirely real and independent of the frequency.

According to [Scharfetter H, Lackner HK, Rosell J. Magnetic induction tomography: Hardware for multi-frequency measurements in biological tissues. Physiol. Meas. 2001; 22: 131 - 146.] a conducting sphere in empty space (radius r = 25 mm, $\sigma$ = 1 S/m and $\varepsilon_r$ = 80) positioned halfway on the axis between two coils (35 mm radius, inter-coil distance 250 mm) produces a $\Delta V/V_0$ of $-1.8*10^{-10}$ - j $8.9*10^{-7}$ at 50 kHz. $|Im(\Delta V/V_0)|$ increases near the sending or receiving coils, but remains between $10^{-5}$ and $10^{-6}$. This means that the wanted signal can easily be by 1 million smaller than the excitation signal. At 10 MHz the value is by a factor of approximately 200 larger, $|Im(\Delta V/V_0)|$ reaching values between $10^{-3}$ and $10^{-4}$. Systems operating at 10 - 20 MHz could thus be realized with simple receiver coils and still a comparatively moderate dynamic range of the signal processing electronics. Low-frequency systems require either Analog-to-Digital Converters with very high dynamic range (>120 dB) or special compensation techniques in order to reduce Vo. So far mostly zero net flux coils have been used by either building gradiometers specially oriented coils which damp the primary signal by factors up to 1000. It should be pointed out that such cancellation techniques do not improve the SNR unless the discretization noise becomes predominant.

The only known true multifrequency system operating below 1 MHz [Scharfetter H., Köstinger A., Issa S. Hardware for quasi-single-shot multifrequency magnetic induction tomography (MIT): the Graz Mk2 system. Physiol. Meas. 2008; 29: S 431 - S 443] has two ring shaped coil arrays with 8 assemblies of a solenoid transmitter coil and a planar Gradiometer as 8 receiver coil on each ring, i. e. 16 assemblies in total. The coils are arranged in a zig-zag pattern around the perimeter. To drive all 16 transmitter coils a 8 to 16 multiplexer is provided. The response voltages from the 16 receiver coils are preamplified and demultiplexed 16 to 8 before being digitized by an 8-channel ADC board. As the 8 transmitter coils are fed simultaneously they must be encoded by splitting the carrier frequencies into 8 closely neighbouring sub-frequencies. The amplitude and phase of each signal is then extracted by using FFT. This parallel-parallel concept allows for extremely fast data acquisition, theoretically one frame can be obtained in 40 ms.

It is also possible to use Scanning systems, whereby a single transmitter / receiver coil is scanned in 2-D above the surface of the measured object. Strictly spoken such a system is not a real tomograph, as it uses only the diagonal elements of the full data matrix but it nevertheless allows image reconstruction.

As analyzed in [Scharfetter H., Casañas R., Rosell J., see above] the usually very small $\Delta V/V$ is susceptible to interference and drift. The most important sources for systematic errors in a zero net flux channel are:

- moving well-conducting and/or ferromagnetic material near the object space
- thermal drifts of the receiver's electrical properties
- displacements of the receiver coil with respect to a transmitter coil
- phase-mismatch between the channels for the reference coil and receicer coils.

Displacements between the transmitter and receiver coils can easily occur simply by natural vibrations as already very minute shifts lead to considerable signals. Theoretically these unwanted signals appear essentially in the real part of $\Delta V/V$ which leads to the fact that measurements of this real part (related to permittivity and permeability) are always much noisier than measurements of the imaginary part. Due to many parasitic effects in the receiver coils and electronics, however, also the imaginary part is usually affected to some extent and a certain part of the vibrational modulations of the real part are projected to the imaginary part. This leads to an unequal distribution of the noise among different transmitter/receiver channels as observed by several authors, e.g. [H. Scharfetter, S. Issa. Reduction of low-frequency noise in magnetic induction tomography systems. Proc. 8th EMBEC, Ant-werp. 2008: 752 - 55]. Typically the noise in combinations with strong magnetic coupling (usually coils in close neighbourhood) is much stronger than in coils with weak coupling. Usually the noise is dominated by very low frequencies (less than 100 Hz), which is an indirect proof that it is caused by mechanical vibrations. WO 2008/011649 A1 suggest a method how to improve the vibrational SNR by up to 30 dB. The basic idea is to intentionally vibrate the coil system with a mechanical actuator and to determine a correction angle by which the complex signal must be rotated with respect to the local oscillator phase so that the standard deviation of the imaginary part is minimized. This method not only improves the SNR but also automatically determines the correct reference angle for all transmitter/receiver combinations, which makes an external reference channel for demodulation unnecessary.

The reconstruction of the absolute conductivity in a target region $\Omega$ requires the solution of a complex inverse eddy current problem. Let

$$\mathbf{y} = \Psi(\kappa) \tag{3}$$

be the discretized non-linear forward mapping of the conductivity vector $\kappa$ to the vector of induced voltages $\mathbf{y}$. $\mathbf{y}$ contains $M = a \times b$ entries, a being the number of TX and b that of RX coils. Depending on the material properties of the object and the frequency the formulation may be simplified. The forward operator $\Psi(\kappa)$ is usually given by quasi-stationary Maxwell's equations for harmonic excitation. If the retardation of the potentials does not play a significant role and only weak perturbations are assumed (medical MIT at low frequencies < 1 MHz) the problem can first be broken down to a reduced vector potential formulation. In medical MIT the problem can even be reduced to the solution of a scalar potential equation which usually is solved with the finite elements method (FEM), e.g. [ Soleimani M. Computational aspects of low frequency electrical and electromagnetic tomography: A review study. International J Num Anal Mod. 2008; 5 (3): 407 - 440.] or the finite differences method (FDM) [Morris A., Griffiths H., Gough W. A numerical model for magnetic induction tomographic measurements in biological tissues. Physiol Meas. 2001; 22: 113 - 119.]. The inverse problem corresponding to (3)

$$\kappa = \Psi^{-1}(\mathbf{y}) \qquad (4)$$

is mathematically ill-posed and usually strongly underdetermined. The generic approach for the solution of this type of non-linear problem is the application of an iterative scheme such as the regularized Gauss-Newton approach, including an appropriate regularization scheme. The solution of (4) requires the target region to be discretized into N voxels. Within each voxel i the assigned component $\kappa i$ of the vector of conductivity $\kappa$ is usually assumed to be constant. Then the optimal estimate $\kappa *$ of the true parameter vector $\kappa$ is found iteratively by solving the minimization problem:

$$\kappa^{*} = arg\ \min_{\kappa}\left((\Psi(\kappa) - \mathbf{y}_m)^T \mathbf{C}_D^{-1}(\Psi(\kappa) - \mathbf{y}_m) + \lambda \kappa^T \mathbf{R}\kappa\right). \qquad (5)$$

$\mathbf{y}_m$ is the measured data vector. $\mathbf{R}$ and $\lambda$ are a regularization matrix and a regularization parameter, respectively, which are required to stabilize the iteration. $\mathbf{C}_D$ is formally a weighting matrix for the data, frequently the noise covariance matrix. There are many possibilities to solve the non-linear problem or also a linearized version of eq. (5). The reconstruction problem is in any case mathematically and computationally demanding. Fast methods based on the solution of the forward model and a so-called adjoint system have been published in [Hollaus K., Magele C., Merwa R., Scharfetter H. Fast calculation of the sensitivity matrix in magnetic induction tomography by tetrahedral edge finite elements and the reciprocity theorem. Physiol Meas. 2004; 25:159 - 68].

One main difficulty is that the space inside of the receiver boundary consists of two quite different subdomains: a conducting and a non-conducting part corresponding to the test object and the surrounding air, respectively. A particular problem is the boundary between the object and the surrounding air, because the eddy currents are confined to flow within the object and the voltages induced in the receiving coils depend strongly on the currents close to this boundary. In practice one is usually interested only in a small perturbation inside a background object (tank, human head or thorax), while actually the 'signal' is essentially produced by the eddy currents close to the surface of the background object. A conducting perturbation with a diameter of 10 % of that of a thorax and a conductivity of twice that of the tank typically yields a signal which is 3 orders of magnitude less than that of the thorax. This in turn means that a small displacement of the background object can induce a much larger change $\Delta V$ than a diagnostically significant perturbation and image reconstruction becomes extremely difficult. Thus, though numerical problems may be overcome, absolute MIT is very difficult.

In contrast, state differential imaging may work much better in the following particular cases:

The perturbation undergoes a periodic conductivity change with a frequency which overlaps only minimally with the dominant spectral components of the background movements. In this case the perturbation can be identified by its dynamic change from difference data between two states (e. g. beating heart inside of a resting thorax). This modality is called "dynamic imaging".

The perturbation shows a typical $\beta$-dispersion, e. g. a frequency contrast. In this case the movement artifact affects the data at both frequencies in approximately the same manner and cancels out as long as the conductivity difference does not change the eddy current distribution in the background significantly. This seems to be the case in typical biological tissues with frequency differences between several tens up to 100 % of frequency change. This modality is called "frequency-differential imaging".

However in state differential imaging still the sensitivity to movement artifacts is very high. It can, however, be filtered out if the movements of the outer object boundary are known. In frequency differential imaging the artifacts are much less pronounced, but the basic requirement is that the body under investigation is the only one which has a frequency dependent conductivity inside the zone of sensitivity. Unfortunately this is practically never the case, because the shielding material of the coils can introduce considerable errors which are hard to calibrate.

Consequently there is probably only one good method to overcome the movement artifacts: The object boundary should be tracked as exactly as possible and this can be achieved either with a camera system or with an array of distance sensors. This concept as illustrated in [Tapp HS, Goss D, et al. A combined digital camera - EMT system to measure human body composition. Proc 3rd World Congress on Industrial Process Tomography. 2003; 384 - 9.] appears attractive but on the other hand it makes the system much more complicated and expensive.

Summary of the invention

It is an object of the present invention to provide a method and an apparatus for MIT which allows eliminating artefacts due to movements of the body under investigation without causing high costs and complicated equipment.

This object is achieved by a method according to the preamble of claim 1 which according to the invention is characterized by placing a set of markers on the surface of the object under investigation, the markers being selected from the group of markers producing a signal which is clearly distinguishable from that of the object, performing measurements to reconstruct the image of the object itself on the one hand and to reconstruct the image and location of the markers on the other hand and filtering out the movements of the outer object boundary in order to eliminate artifacts due to such movements.

A variant of the invention is characterized by placing a set of markers on the surface of the object under investigation, the markers being selected from the group of "real part modulators", i. e. markers producing an essentially pure real signal and performing a measurement and synchronously demodulating the signal so as to separate into real and imaginary part, using the real part for reconstructing the markers and using the imaginary part for reconstructing the image of the object.

A further variant is characterized by placing a set of markers on the surface of the object under investigation, the markers being selected from the group of "active markers", the markers affecting the real and/or the imaginary of the signal performing a first measurement under a first electric condition and building a first data set from the signals received, performing a second measurement under a second electric condition and building a second data set from the signals received, the first electric condition and the second electric condition causing appreciable different signal levels induced by the markers, and subtracting the data sets from each other for the reconstruction of the markers and using the data set without marker signal for image reconstruction

In a recommended variant with a view to make the markers totally invisible in the signal ,it is provided that each marker consist of a loop, the wire being electrically conducting, and a remotely controlled switch, adapted to open and close the loop, performing the first measurement with the switches of all markers open and building the first data set from the signals received, performing the second measurement with the switches of all markers closed and building the second data set from the signals received, and subtracting the data sets from each other for the reconstruction of the markers while using the first data set for the reconstruction of the object.

If at least two transmitter coils are actuated simultaneously it is advantageous that each excitation frequency is split up into several closely spaced sub-frequencies, wherein the closely neighboring sub-frequencies deviate from each other only insignificantly with respect to the frequency dependence of the passive electrical properties of the target tissue. In this case it is recommendable that the number of transmitter coils corresponds to the number of sub-frequencies per excitation frequency and each first, second, third etc. transmitter coil is fed with the first, second, third etc. sub-frequency of the excitation frequency.

The object is also achieved with an apparatus for magnetic induction tomography comprising at least one transmitter coil for the introduction of an alternating magnetic field into a target body to be investigated, at least one receiving coil for the pick-up of received signals and means for processing the received signals which reconstructs an image of the spatial electrical properties in the object from the amplitudes and phases of the received signals, characterized by a set of markers on the surface of the object under investigation, the markers being selected from the group of markers producing a signal which is clearly distinguishable from that of the object.

Hereby, in a preferred embodiment, each marker is an active marker consisting of a loop, the wire being electrically conducting, and a remotely controlled switch, adapted to open and close the loop.

It is further advantageous if all markers are arranged on a flexible belt adapted to be affixed at the surface of the object under investigation. Using such a belt accelerates the preparing procedure in MIT.

Brief description of the drawings

The specific features and advantages of the present invention will become better understood with regard to the following description. In the following, the present invention is described in more detail with reference to the drawings, which show:

Fig. 1 illustratively and schematically the principle of MIT,

Fig. 2 in a block diagram the principle of a measurement arrangement in accordance with the state of the art,

Fig. 3 schematically how translatory movements as well as deformations of the object boundary can be traced,

Fig. 4 a marker array in form of a flexible belt,

Fig. 5 a block diagram the principle of a measurement arrangement in accordance with the present invention and

Fig. 6 in a flow chart an example of the method according to the present invention.

Detailed description of the invention

As also explained in the introduction additional sensors are, in general, undesirable and thus it would be a great advantage to get the tracking information from the MIT sensors (the coils). This is, in principle, possible, because only half of the entire information is usually used for image reconstruction. Due to technical reasons the real part of the relative voltage changes is usually much more contaminated by noise and drifts than the imaginary part [Scharfetter H., Casañas R., Rosell J., see above]. Therefore mostly only the imaginary part (or, in some other applications, only the phase information) is used for image reconstruction.

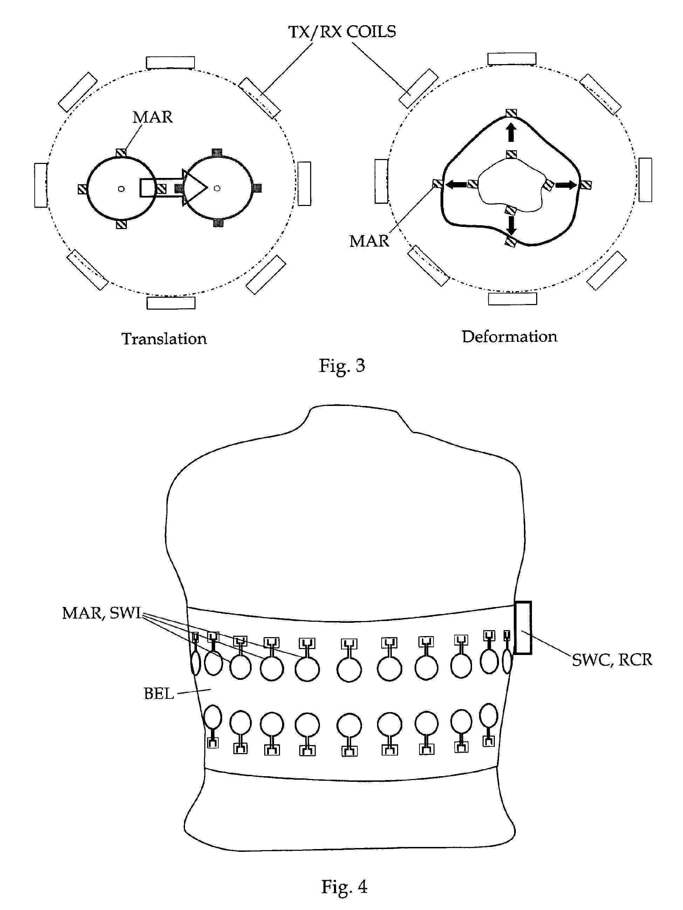

The basic idea now is to place on the surface of the body under investigation a set of strategically placed markers which, under a certain condition, produce a signal which is clearly distinguishable from that of the object and which, in turn, does not perturb the signal of the object. There are two basic types of markers: Passive markers which affect only the real part of the signal and Active markers which can be switched on and off so that they become either visible or invisible for the field. The idea is illustrated in Fig. 3 where on can see that translatory movements as well as deformations of the object boundary provided with markers MAR can be traced when imaging the locations of sufficiently closely spaced markers on the surface of the object.

Passive markers are so called real part modulators, i. e. they produce a strong signal in the real part but virtually no signal in the imaginary part. In that way they would not perturb the tissue image but their position could be reconstructed from the real part data.

Important conditions are:

a) The markers produce a practically purely real signal

b) The marker signal is sufficiently stronger than the noise in the real part.

Either purely magnetic, but non-conducting material, highly conducting but non-magnetic materials or materials with extremely high permittivity are potential candidates for markers. Ideally they should produce pure real parts and shall therefore be called real part modulators (RPM). As no practically available material possesses these properties in ideal form, there will always be some imaginary part associated with the marker. Reasons are dissipative losses, and in order to allow for a correct image reconstruction the imaginary contribution must be by at least two orders of magnitude lower than the signal which is produced by the tissue under investigation. On the other hand the real part should be so large that a good SNR for tracking is achieved. In order to suppress the influence on the imaginary part further, the reference phase angle of the synchronous demodulator can be corrected in such a way that the influence of the markers on the imaginary part is minimized. One possibility for achieving this phase correction is the method described in WO 2008/011649 A1. Very briefly the problem to be solved in this publication was to suppress artifacts in the imaginary part which are due to vibrational movements of the coils relative to each other. Theoretically such vibrations modulate only the real part of the received signals, but due to several phase shifts in the receiver chain the modulations are also projected into the imaginary part, hence causing significant noise. The suggested solution is to intentionally introduce mechanical vibrations into the coil system during a calibration cycle and to turn the reference phase of the local oscillator in such a way that the modulations only project to the real part.

As real part modulators also essentially produce a real part signal, movements of the markers fulfill nearly the same phase correction condition as mechanical vibrations. Thus the same correction method can be used if, instead of the coils, the markers are moved inside the coil system during the calibration cycle. Minimizing the modulations of the imaginary part will then make the markers essentially invisible in the imaginary part, even if they behave in a slightly non-ideal manner. The movements need not be introduced by a dedicated mechanism because already the natural movements of the measured subject would suffice to give a strong calibration signal.

Accordingly, in one embodiment, the invention provides to place a set of markers on the surface of the object OBJ under investigation, the markers being selected from the group of "real part modulators", i. e. markers producing an essentially pure real signal, and then to perform a synchronous demodulation in order to separate the marker signal from the object signal, the marker signal being used for the determination of the object boundary and the object signal being used for image reconstruction.

Although there are some passive materials which yield fairly low imaginary parts, usually their influence on the wanted signal is still high and the phase correction algorithm in many cases is not able to suppress it sufficiently. However in some cases, due to their simplicity, passive markers may be used.

Accordingly another embodiment of the invention provides to use active markers and to perform a first measurement under a first electric condition, building a first data set from the signals received, then to perform a second measurement under a second electric condition, building a second data set from the signals received. Naturally it is essential that the first electric condition and the second electric condition cause appreciably different signal levels induced by the markers. In particular the markers should produce no signal under one of both conditions and either a real, an imaginary or both signals under the other condition. Then the data sets are subtracting from each other for the reconstruction of the marker locations while the object signal is contained in that data set in which the markers give no contribution.

For the tracking and filtering it is assumed that repetitive measurements are taken so fast that the object movement between two subsequent measurements can be considered as small enough to allow a reliable reconstruction of the marker trajectories (Nyquist-criterion: sampling frequency must be lower than half of the maximum significant frequency of the marker signal). Then the markers are reconstructed by solving the inverse problem eq. (4) from the marker signals. The changes of the object boundary are then obtained by interpolating the reconstructed changes of the marker positions. Afterwards the object is reconstructed with eq. (5) from the object signal under consideration of the boundary changes.

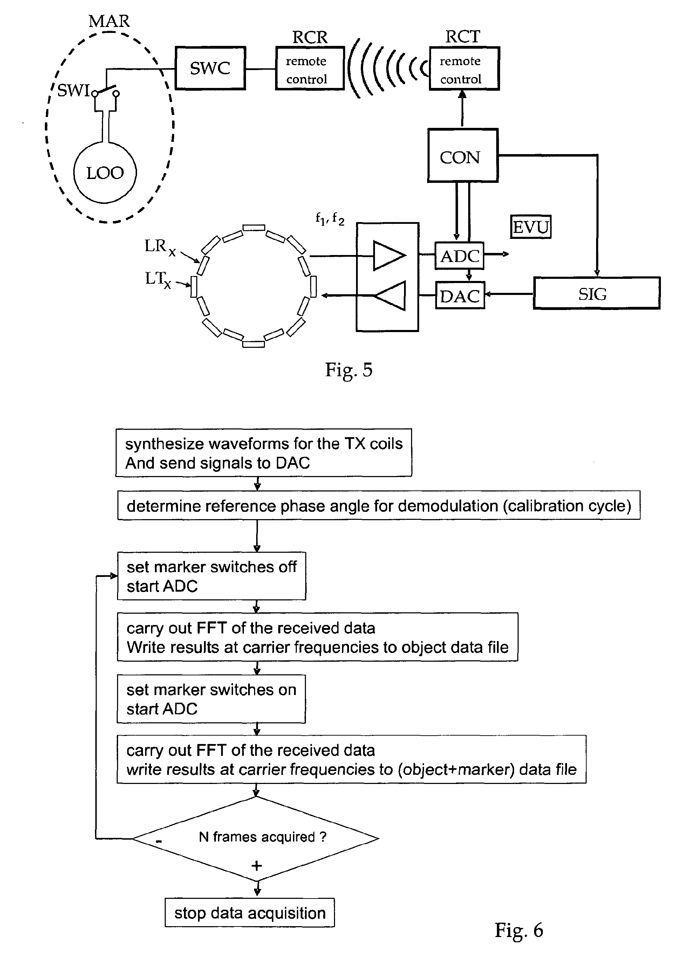

Figs. 4 and 5 show that advantageously active markers may be used which consist of a small loop LOO of a very thin wire which can be opened and shorted via a tiny switch SWI. When the loop is open it does not allow eddy currents to flow and therefore it is invisible in the reconstructed image. When the switch SWI is closed, strong eddy currents flow and the tracking signal is obtained. The switch must be mounted in a zone of low sensitivity of the coils so that it does not contribute to the formation of eddy currents. Possible realizations are SMD FETs the gates of which are controlled remotely, but also other switches can be thought of. The switches must be controlled synchronously to the data acquisition protocol, e. g. in such a way that every frame is acquired twice, once with the switch closed and once with the switch opened. Fiber optic cables may be used to send the switching signals to the individual switches associated to the loops. Such cables will nearly not contribute to the generation of the picture. An example is illustrated in Figs. 4 and 5, where a belt BEL with a set of several such markers MAR including switches SWI is shown. There is also shown a switch control SWC and a remote control receiver RCR.

The block diagram of Fig. 5 likewise explains the principle of a measurement arrangement in accordance with the present invention. Transmitter and receiver coils are arranged around an object (not sown here) and are connected to a RX/TX-unit RX/TX. The transmitter coils are excited as per example with two frequencies $f_1$ and $f_2$ but even more frequencies may be used as explained below. A MIT control unit CON is used to control the whole procedure and is connected with A/D and D/A converters ADC and DAC, with the signal generator SIG and with an evaluation unit EVU, comparable with units SYD and BIR in Fig. 2.

Fig. 6 is a flow chart an example of the method according to the present invention which by way of example shows acquiring and acquisition ob object data and markers data, alternately with switches SWI opened and closed. As explained above the markers are reconstructed by solving the inverse problem from the marker signals. Thereafter the changes of the object boundary are obtained by interpolating the reconstructed changes of the marker positions. Afterwards the object is reconstructed from the object signal under consideration of the boundary changes.

A rapid and precise imaging is substantially promoted by the simultaneous excitation of many, if not all the coils. For the case of multi-frequency imaging all the frequencies, e. g. $f_1$ and $f_2$, should be used simultaneously to avoid any drift between the measurements at different frequencies. However, if several coils are excited simultaneously at the same frequency, the imaging fails since the superposed individual contributions can no longer be separated from one another.

This problem may be solved as explained more detailed in WO 2008/011649 A1: the various frequencies to be used can be split, usually by a few tenths of a percent. Thus, the n different transmitter $L_{TX}$ coils can be marked by splitting the excitation frequencies $f_1$ and $f_2$ into n-tuple closely spaced frequencies (multiple-carrier concept). As far as the choice of the frequency interval is concerned, this must be selected so that on the one hand it still allows the separation of individual excitation signals, e. g. by synchronous rectification (e. g. 1 kHz) and on the other hand, the conductivity of the target object can be assumed to be constant within the bandwidth of the resulting sub-carrier packets.

## Claims

1. Method for magnetic induction tomography, where an object (OBJ) having inhomogeneous electrical properties is subjected to alternating magnetic fields with at least one excitation frequency by means of at least one transmitter coil (ES1, ES2, ES3), picking up alternating voltage signals which contain information on the electrical conductivity and its distribution in the object by means of at least one receiving coil (SP1, SP2, SP3), electrically ore mechanically locating the at least one receiving coil at different reception locations and reconstructing an image of the electrical properties within the object from the received signals, having an imaginary and a real part, by using their different phases and amplitudes,

**characterized by**
placing a set of markers on the surface of the object (OBJ) under investigation, the markers being selected from the group of markers producing a signal which is clearly distinguishable from that of the object, performing measurements to reconstruct the image of the object (OBJ) itself on the one hand and to reconstruct the image and location of the markers (MAR) on the other hand and filtering out the movements of the outer object boundary in order to eliminate artifacts due to such movements.

2. Method according to claim 1 **characterized by** placing a set of markers on the surface of the object (OBJ) under investigation, the markers being selected from the group of "real part modulators", i. e. markers producing an essentially pure real signal and performing a measurement and synchronously demodulating the signal so as to separate into real and imaginary part, using the real part for reconstructing the markers and using the imaginary part for reconstructing the image of the object.

3. Method according to claim 1 **characterized by** placing a set of markers on the surface of the object (OBJ) under investigation, the markers being selected from the group of "active markers", the markers affecting the real and/or the imaginary of the signal
performing a first measurement under a first electric condition and building a first data set from the signals received,
performing a second measurement under a second electric condition and building a second data set from the signals received,
the first electric condition and the second electric condition causing appreciable different signal levels induced by the markers, and
subtracting the data sets from each other for the reconstruction of the markers and using the data set without marker signal for image reconstruction

4. Method according to claim 3, wherein each marker consist of a loop, the wire being electrically conducting, and a remotely controlled switch, adapted to open and close the loop, performing the first measurement with the switches of all markers open and building the first data set from the signals received, performing the second measurement with the switches of all markers closed and building the second data set from the signals received, and subtracting the data sets from each other for the reconstruction of the markers while using the first data set for the reconstruction of the object.

5. Method according to any of claims 1 to 4, wherein at least two transmitter coils are actuated simultaneously **characterized by** the fact that each excitation frequency (f1, f2) is split up into several closely spaced sub-frequencies, wherein the closely neighboring sub-frequencies deviate from each other only insignificantly with respect to the frequency dependence of the passive electrical properties of the target tissue.

6. Method according to claim 5, wherein the number of transmitter coils corresponds to the number of sub-frequencies per excitation frequency and each first, second, third etc. transmitter coil is fed with the first, second, third etc. sub-frequency of the excitation frequency.

7. Apparatus for magnetic induction tomography comprising at least one transmitter coils (ES1, ES2, ES3) for the introduction of an alternating magnetic field into a target body (OBJ) to be investigated, at least one receiving coil (SP1, SP2, SP3) for the pick-up of received signals and means (STE, SYD, BIR) for processing the received signals which reconstructs an image of the spatial electrical properties in the object from the amplitudes and phases of the received signals, **characterized by** a set of markers (MAR) on the surface of the object (OBJ) under investigation, the markers (MAR) being selected from the group of markers producing a signal which is clearly distinguishable from that of the object.

8. Apparatus for magnetic induction tomography according to claim 7, wherein each marker (MAR) is an active marker consisting of a loop (LOO), the wire being electrically conducting, and a remotely controlled switch (SWI), adapted to open and close the loop.

9. Apparatus for magnetic induction tomography according to claim 8 with a switch control (SWC) adapted to open and close all switches of the set simultaneously.

10. Apparatus for magnetic induction tomography according to any of claims 7 to 9, whereby all markers are arranged on a flexible belt (BEL) adapted to be affixed at the surface of the object (OBJ) under investigation.

Fig. 1

primary field
response field

Fig. 2

TX/RX COILS

MAR

MAR

Translation

Deformation

Fig. 3

MAR, SWI

BEL

SWC, RCR

Fig. 4

Fig. 5

synthesize waveforms for the TX coils
And send signals to DAC

determine reference phase angle for demodulation (calibration cycle)

set marker switches off
start ADC

carry out FFT of the received data
Write results at carrier frequencies to object data file

set marker switches on
start ADC

carry out FFT of the received data
write results at carrier frequencies to (object+marker) data file

N frames acquired ?

－

＋

stop data acquisition

Fig. 6

Europäisches Patentamt
European Patent Office
Office européen des brevets

**EUROPÄISCHER RECHERCHENBERICHT**

Nummer der Anmeldung

EP 09 45 0236

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2008/194982 A1 (LANFERMANN GERD [DE] ET AL) 14 August 2008 (2008-08-14) | 1,3,4, 7-10 | INV.<br>A61B5/053<br>A61B5/11<br>A61B19/00 |
| Y | * paragraphs [0004], [0008], [0013] - [0028] *<br>* paragraphs [0035] - [0043]; figure 2 *<br>* paragraphs [0045] - [0047]; figures 5,6 * | 2,5,6 | |
| | ----- | | |
| X | GÜRSOY D, SCHARFETTER H: "Reconstruction artefacts in magnetic induction tomography due to patient's movement during data acquisition"<br>PHYSIOLOGICAL MEASUREMENT, INSTITUTE OF PHYSICS PUBLISHING, BRISTOL, GB,<br>vol. 30, no. 6, 1 June 2009 (2009-06-01), pages S165-S174, XP020161081<br>ISSN: 0967-3334<br>* abstract *<br>* Section 2.7 *<br>* Section 4 * | 1,7 | |
| | ----- | | **RECHERCHIERTE SACHGEBIETE (IPC)** |
| Y,D | CASANAS R ET AL: "Biological tissue characterization by magnetic induction spectroscopy (MIS): requirements and limitations"<br>IEEE TRANSACTIONS ON BIOMEDICAL ENGINEERING, IEEE SERVICE CENTER, PISCATAWAY, NJ, US LNKD-DOI:10.1109/TBME.2003.813533,<br>vol. 50, no. 7, 1 July 2003 (2003-07-01), pages 870-880, XP011097445<br>ISSN: 0018-9294<br>* Section B.1); page 876 * | 2 | A61B |
| | ----- | | |
| Y,D | WO 2008/011649 A1 (UNIV GRAZ TECH [AT]; FORSCHUNGSHOLDING TU GRAZ GMBH [AT]; SCHARFETTER) 31 January 2008 (2008-01-31)<br>* page 3, paragraph 4-5 * | 5,6 | |
| | ----- | | |
| | -/-- | | |

Der vorliegende Recherchenbericht wurde für alle Patentansprüche erstellt

| Recherchenort | Abschlußdatum der Recherche | Prüfer |
|---|---|---|
| Berlin | 20 May 2010 | Kronberger, Raphael |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**Europäisches Patentamt**
**European Patent Office**
**Office européen des brevets**

# EUROPÄISCHER RECHERCHENBERICHT

Nummer der Anmeldung

EP 09 45 0236

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 2009/144461 A2 (UGCS UNIVERSITY OF GLAMORGAN C [GB]; WATSON STUART [GB]) 3 December 2009 (2009-12-03) * page 7, line 7 - page 9, line 22 * * page 12, line 25 - page 13, line 18; figure 5 * * page 17, lines 6-17 * * page 18, line 26 - page 19, line 8 * ----- | 1,7 | |
| A | WO 2009/149409 A1 (CALYPSO MED TECHNOLOGIES INC [US]; PARIKH PARAG J [US]; DIMMER STEVEN) 10 December 2009 (2009-12-10) * paragraphs [0002], [0003], [0011], [0019] - [0021] * ----- | 3 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| Berlin | 20 May 2010 | Kronberger, Raphael |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**            EP 09 45 0236

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-05-2010

| Im Recherchenbericht angeführtes Patentdokument | Datum der Veröffentlichung | Mitglied(er) der Patentfamilie | Datum der Veröffentlichung |
|---|---|---|---|
| US 2008194982 A1 | 14-08-2008 | CN 101203174 A<br>WO 2006137012 A2<br>JP 2008546465 T | 18-06-2008<br>28-12-2006<br>25-12-2008 |
| WO 2008011649 A1 | 31-01-2008 | AT 504060 A1<br>CN 101517436 A<br>EP 2044470 A1 | 15-02-2008<br>26-08-2009<br>08-04-2009 |
| WO 2009144461 A2 | 03-12-2009 | GB 2462243 A | 03-02-2010 |
| WO 2009149409 A1 | 10-12-2009 | KEINE | |

Für nähere Einzelheiten zu diesem Anhang : siehe Amtsblatt des Europäischen Patentamts, Nr.12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008011649 A1

### Non-patent literature cited in the description

- **GRIFFITHS H.** Magnetic induction tomography. *Meas. Sci. Technol.,* vol. 26, 1126-1131
- **KORZHENEVSKII A. V. ; V. A. CHEREPENIN.** Magnetic induction tomography. *J. Commun. Tech. Electron.,* 1997, vol. 42, 469-474
- **HERMANN SCHARFETTER ; ROBERTO CASANAS ; JAVIER ROSELL.** Biological Tissue Characterization by Magnetic Induction Spectroscopy (MIS): Requirements and Limitations. *IEEE Trans. Biomed. Eng.,* 2003, vol. 50, 870-880
- **MORTARELLI J. R. A.** Generalization of the Geselowitz Relationship Useful in Impedance Plethysmographic Field Calculations. *IEEE Trans. Biomed. Eng.,* 1980, vol. 27, 665-667
- **GENCER NG ; TEK MN.** Electrical conductivity imaging via contactless measurements. *IEEE Trans Med Imag.,* 1999, vol. 18, 617-27
- *IEEE Trans Biomed Eng.,* 2003, vol. 50, 870-80
- **SCHARFETTER H ; LACKNER HK ; ROSELL J.** Magnetic induction tomography: Hardware for multi-frequency measurements in biological tissues. *Physiol. Meas.,* 2001, vol. 22, 131-146
- **SOLEIMANI M.** Computational aspects of low frequency electrical and electromagnetic tomography: A review study. *International J Num Anal Mod.,* 2008, vol. 5 (3), 407-440
- **MORRIS A. ; GRIFFITHS H. ; GOUGH W.** A numerical model for magnetic induction tomographic measurements in biological tissues. *Physiol Meas.,* 2001, vol. 22, 113-119
- **HOLLAUS K. ; MAGELE C. ; MERWA R. ; SCHARFETTER H.** Fast calculation of the sensitivity matrix in magnetic induction tomography by tetrahedral edge finite elements and the reciprocity theorem. *Physiol Meas.,* 2004, vol. 25, 159-68
- **TAPP HS ; GOSS D et al.** A combined digital camera - EMT system to measure human body composition. *Proc 3rd World Congress on Industrial Process Tomography,* 2003, 384-9